Europäisches Patentamt

European Patent Office

Office européen des brevets

(11) Veröffentlichungsnummer: **0 210 518**
A2

## (12) EUROPÄISCHE PATENTANMELDUNG

(21) Anmeldenummer: 86109599.0

(22) Anmeldetag: 14.07.86

(51) Int. Cl.⁴: **C 07 C 45/51**
C 07 C 47/575, C 07 C 45/59
C 07 C 43/313, C 07 C 43/315
C 07 C 41/56, C 07 C 41/52
C 07 D 317/16, C 07 D 317/22

(30) Priorität: 27.07.85 DE 3526930

(43) Veröffentlichungstag der Anmeldung:
04.02.87 Patentblatt 87/6

(84) Benannte Vertragsstaaten:
CH DE FR GB IT LI NL

(71) Anmelder: Haarmann & Reimer GmbH
Postfach 1253
D-3450 Holzminden(DE)

(72) Erfinder: Finkelmeier, Horst, Dr.
Auf dem Gehrenkamp 6
D-3450 Holzminden(DE)

(72) Erfinder: Hopp, Rudolf, Dr.
Auf dem Gehrenkamp 28
D-3450 Holzminden(DE)

(74) Vertreter: Schumacher, Günter, Dr. et al,
c/o Bayer AG Konzernverwaltung RP Patentabteilung
D-5090 Leverkusen 1 Bayerwerk(DE)

(54) Verfahren zur Herstellung von o- und p-Alkoxy-benzaldehyden.

(57) Die Erfindung betrifft ein neues Verfahren zur Herstellung von o-bzw. p-Alkoxy-benzaldehyden; gemäß diesem Verfahren wird

a) o- bzw. p-Chlor-benzaldehyd in an sich bekannter Weise mittels eines einwertigen Alkohols, eines Diols oder eines Orthoesters in ein o- bzw. p-Chlor-benzaldehyd-acetal.

b) das o- bzw. p-Chlor-benzaldehyd-acetal durch Umsetzen mit einem Natrium- oder Kaliumalkoholat in einem aprotischen, polaren organischen Lösungsmittel bei erhöhten Temperaturen in ein o- bzw. p-Alkoxy-benzaldehyd-acetal und

c) dieses o- bzw. p-Alkoxy-benzaldehyd-acetal in an sich bekannter Weise durch sauer katalysierte Hydrolyse oder Umacetalisierung in den entsprechenden o- bzw. p-Alkoxy-benzaldehyd überführt.

EP 0 210 518 A2

Haarmann und Reimer GmbH    Holzminden

B/m-c

Verfahren zur Herstellung von o- und p-Alkoxy-benzaldehyden

Die Erfindung betrifft ein neues Verfahren zur Herstellung
von o- und p-Alkoxy-benzaldehyden.

o- und p-Alkoxy-benzaldehyde, unter ihnen insbesondere der
Anisaldehyd (p-Methoxybenzaldehyd) sind wichtige Riech-
und Aromastoffe. Außerdem sind sie wichtige Zwischenprodukte für die Herstellung von Zimtsäureestern, die ihrerseits als UV-Filter in Sonnenschutzpräparaten verwendet
werden. Wegen der Bedeutung der o- und p-Alkoxy-benzaldehyde wurde intensiv an der Ausarbeitung geeigneter Herstellungsverfahren gearbeitet und die verschiedensten
Herstellungsverfahren entwickelt.

So wurde zur Herstellung der o- bzw. p-Alkoxy-benzaldehyde
die Oxidation von o- bzw. p-Alkoxy-methyl-benzolen vorgeschlagen. Gemäß DE-PS 27 57 031 dient als Oxidationsmittel
Sauerstoff, gemäß US-PS 4 212 717 Metalloxide wie Mangandioxid. In anderen Verfahren werden Metallsalze, in denen

HR 104 -Ausland

ein Übergangsmetall in einer höheren Oxidationsstufe vorliegt, eingesetzt; gemäß DE-OS 30 28 758 zum Beispiel Cer(IV)-ammoniumnitrat oder gemäß Rozniki Chemii 47, 43 (1933) Cobalt(III)-Salze. In der US-PS 4 354 904 wird die elektrochemische Oxidation beschrieben und in der DE-OS 22 21 116 die Oxidation mit Alkaliperoxidisulfaten in Gegenwart von Silbersalzen.

Alle diese Oxidationsverfahren haben jedoch den Nachteil, daß sie nicht selektiv verlaufen und daß deshalb neben den gewünschten Aldehyden die entsprechenden Alkohole und Carbonsäuren in teilweise erheblichen Mengen entstehen.

Als andere wichtige Reaktion zur Herstellung von o- bzw. p-Alkoxy-benzaldehyden wird ferner die Formylierung von Alkoxy-benzolen vorgeschlagen; diese Formylierung wurde nach verschiedenen Methoden vorgenommen. So ist in Bull. Soc. Chim. France 1955, 1594 die Vilsmeier-Formylierung mit Phosphoroxychlorid und Dimethylformamid und in der Japan. Patentanmeldung 76 26 836 (referiert in C.A. 85, 94067g) mit Phosphoroxychlorid und N-Methylformanilid beschrieben. In J. Chem. Soc. 1932, 2793 ist die Gattermann-Synthese mit Blausäure und Aluminiumchlorid und in Amer. Perfumer Aromatics 69, 31 (1957) die Gattermann-Koch-Synthese mit Kohlenmonoxid und Chlorwasserstoff in Gegenwart von Aluminiumchlorid beschrieben.

Diese Formylierungsreaktionen haben jedoch den Nachteil, daß sie die gewünschten Aldehyde nur in unbefriedigenden Ausbeuten liefern. Außerdem werden stets Gemische von o-

HR 104

und p-Alkoxy-benzaldehyden erhalten; die Trennung dieser Gemische in o- und p-Isomere ist sehr aufwendig.

Als weitere Reaktion zur Herstellung von o- und p-Alkoxy-benzaldehyden wurde die Ozonolyse von (Alkoxy-phenyl)-olefinen vorgeschlagen. Das bekannteste Beispiel für diesen Reaktionstyp ist die Herstellung von Anisaldehyd durch Ozonolyse von Anethol (siehe US-PS 2 916 499). Wegen des hohen Preises der (Alkoxy-phenyl)-olefine, z.B. des Anethols, ist dieses Verfahren jedoch unwirtschaftlich.

Weiterhin wurde zur Herstellung von o- und p-Alkoxy-benzaldehyden vorgeschlagen, Salicylaldehyd bzw. p-Hydroxybenzaldehyd mit Alkylhalogeniden oder Dialkylsulfaten in Gegenwart wäßriger Alkalilauge zu verethern (siehe Chem. Ber. 10, 63 (1877)). Aber auch dieses Verfahren ist für die Herstellung der o- und p-Alkoxy-benzaldehyde in technischem Maßstab ungeeignet, da die als Ausgangsverbindungen benötigten Hydroxy-benzaldehyde nur nach aufwendigen Verfahren in schlechten Ausbeuten erhältlich sind.

Als weiteres Herstellungsverfahren für o- und p-Alkoxy-benzaldehyde wurde ferner die Chlormethylierung von Alkoxy-benzolen mit Formaldehyd und Chlorwasserstoff und die nachfolgende Umsetzung der so erhaltenen o- bzw. p-Chlormethyl-alkoxy-benzole mit Hexamethylentetramin in der US-PS 4 058 566 beschrieben. Dieses Verfahren ist jedoch für eine Herstellung in industriellem Maßstab ungeeignet, da bei dieser Reaktion als Nebenprodukt der stark krebs-erregende Dichlor-dimethylether entsteht.

HR 104

Überraschenderweise wurde nun gefunden, daß man auf einfache Weise in hohen Ausbeuten o- und p-Alkoxy-benzaldehyde aus gut zugänglichen Ausgangsverbindungen herstellen kann, wenn man o- bzw. p-Chlorbenzaldehyd in an sich bekannter Weise in ein o- bzw. p-Chlorbenzaldehyd-acetal überführt, in diesem durch Umsetzung mit Alkalialkoholaten das Chloratom gegen eine Alkoxygruppe austauscht und das erhaltene Alkoxy-benzaldehyd-acetal durch Spaltung der Acetalgruppe in den freien o- bzw. p-Alkoxy-benzaldehyd überführt. Nach diesem Verfahren werden o- bzw. p-Alkoxy-benzaldehyde, z.B. Anisaldehyd, mit hoher Selektivität und in hoher Reinheit erhalten.

Die Erfindung betrifft daher ein neues Verfahren zur Herstellung von o- bzw. p-Alkoxy-benzaldehyden, das dadurch gekennzeichnet ist, daß man

1. o- bzw. p-Chlorbenzaldehyd in an sich bekannter Weise mittels eines einwertigen Alkohols, eines Diols oder eines Orthoesters in ein o- bzw. p-Chlor-benzaldehyd-acetal überführt;

2. dieses o- bzw. p-Chlor-benzaldehyd-acetal durch Umsetzung mit Natrium- oder Kaliumalkoholat in einem aprotischen polaren organischen Lösungsmittel bei erhöhten Temperaturen in das entsprechende o- bzw. p-Alkoxy-benzaldehyd-acetal überführt und

3. dieses o- bzw. p-Alkoxy-benzaldehyd-acetal in an sich bekannter Weise durch saure Hydrolyse oder Umacetalisieren in den entsprechenden o- bzw. p-Alkoxy-benzaldehyd umwandelt.

HR 104

Die in dem erfindungsgemäßen Verfahren als Ausgangsprodukte verwendeten Chlor-benzaldehyde sind preiswerte und leicht zugängliche Chemikalien, die durch Chlorierung von o-Chlor-toluol bzw. p-Chlor-toluol und anschließende Hydrolyse in großtechnischem Maßstab hergestellt werden.

Die Acetalisierung des o- bzw. p-Chlor-benzaldehyds kann mit einwertigen Alkoholen, Diolen oder Orthoestern vorgenommen werden.

Die einwertigen Alkohole können primäre oder sekundäre Alkohole sein und 1 bis 12 Kohlenstoffatome enthalten. Bevorzugt werden primäre $C_4$-$C_8$-Alkohole wie n-Butanol, i-Butanol, n-Pentanol, 3-Methyl-butanol, n-Hexanol und 2-Ethyl-hexanol verwendet.

Die Diole können 1,2-Diole, 1,3-Diole oder 1,4-Diole mit 1 bis 12 Kohlenstoffatomen sein. Als Beispiele solcher Diole seien genannt: Ethylenglykol, 1,2-Propylenglykol, 1,3-Propylenglykol, 1,4-Butandiol, 2,3-Butandiol und 1,2-Octandiol; bevorzugt wird Ethylenglykol verwendet.

Orthoester eignen sich vor allem zur Herstellung der niederen Dialkylacetale, z.B. des o- bzw. p-Chlor-benzaldehyd-dimethyl- oder -diethylacetals. Bevorzugt werden Orthoameisensäuremethyl- und -ethylester eingesetzt.

Die Acetalisierung des o- bzw. p-Chlor-benzaldehyds mit einwertigen Alkoholen oder Diolen wird in der Weise vor-

HR 104

- 6 -

genommen, daß einem Chlor-benzaldehyd und Alkohol in einem unter den Reaktionsbedingungen inerten organischen Lösungsmittel in Gegenwart katalytischer Mengen Mineralsäure, z.B. Schwefelsäure, Phosphorsäure oder p-Toluolsulfonsäure erhitzt und das bei der Reaktion entstehende Wasser azeotrop mit dem Lösungsmittel abdestilliert wird. Als Lösungsmittel werden vorzugsweise mit Wasser nicht mischbare Lösungsmittel, z.B. Kohlenwasserstoffe wie Benzol, Toluol, Xylole oder Cyclohexan oder halogenierte Kohlenwasserstoffe wie Chloroform, Tetrachlorkohlenstoff oder Chlorbenzol verwendet.

Die Acetalisierung des o- bzw. p-Chlor-benzaldehyds mit Orthoestern wird in der in Organikum 11. Auflage (1972) S. 430 allgemein beschriebenen Verfahrensweise durch Erhitzen des Chlor- benzaldehyds mit dem Orthoester und Alkohol unter Zusatz von Ammoniumnitrat vorgenommen.

Die zur Umsetzung der o- bzw. p-Chlor-benzaldehyd-acetale verwendeten Natrium- oder Kaliumalkoholate leiten sich vorzugsweise von primären oder sekundären aliphatischen $C_1$-$C_{20}$-Alkoholen oder einem gegebenenfalls substituierten Benzylalkohol ab. Bevorzugt werden die Natriumalkoholate primärer $C_1$-$C_4$-Alkohole, insbesondere Natriummethylat, verwendet.

Als Lösungsmittel für die erfindungsgemäße Umsetzung der o- bzw. p-Chlor-benzaldehyd-acetale mit Natrium- bzw. Kaliumalkoholaten eignen sich aprotische, polare organi-

HR 104

sche Lösungsmittel wie N,N-Dimethylformamid, N,N-Dimethylacetamid, Tetramethylensulfon, Dimethylsulfoxid und N-alkylierte Lactame wie N-Methyl-pyrrolidon, N-Methyl-piperidon und N-Methyl-caprolactam. Bevorzugt werden Dimethylsulfoxid und N-Methyl-pyrrolidon verwendet.

Die erfindungsgemäße Umsetzung der o- bzw. p-Chlor-benzaldehyd-acetale mit Natrium- oder Kaliumalkoholat wird bei Temperaturen von 60 bis 170°C, vorzugsweise 80 bis 140°C durchgeführt. Um einen möglichst hohen Umsatz der Chlor-benzaldehyd-acetale zu erreichen, ist es vorteilhaft, ein Molverhältnis von Alkoholat/Chlor-benzaldehyd-acetal von mindestens 1:1, vorzugsweise von 1-1,5:1 anzuwenden. Die Reaktionszeit hängt von der Art des Acetals (Dialkylacetal oder Dioxolan) und des Alkoholats (Natrium- oder Kaliumalkoholat), von der Stellung des Chlors im Chlor-benzaldehyd (o- oder p-Stellung) und vom Lösungsmittel ab. Die Reaktionszeiten betragen etwa 2 bis 36 h, vorzugsweise 10 bis 24 h.

Der letzte Schritt des erfindungsgemäßen Verfahrens, die Spaltung der o- bzw. p-Alkoxy-benzaldehyd-acetale wird durch Hydrolyse mit wäßrigen Mineralsäuren (siehe Houben-Weyl, Methoden der organischen Chemie, IV. Auflage, Band 7/1, S. 424) oder durch sauer katalysierte Umacetalisierung mit anderen Carbonylverbindungen wie Aceton (siehe Synthesis 1984, 1021) durchgeführt.

Die Umsetzung von gegebenenfalls durch inerte Substituenten, wie Alkyl- und Alkoxygruppen substituierten Chlor-

HR 104

benzolen mit Alkoholaten in aprotischen, polaren organischen Lösungsmitteln, bei der das Chloratom gegen eine Alkoxygruppe ausgetauscht wird, ist bekannt (siehe DE-OS 2 210 254). Die Umsetzung von im Kern chlorierten Acetalen aromatischer Aldehyde, wie der o- bzw. p-Chlor-benzaldehyd-acetale, ist jedoch neu. Überraschenderweise gelingt diese Reaktion mit hoher Selektivität, obwohl sich weder o- bzw. p-Chlor-benzaldehyd noch o- bzw. p-Chlor-benzylalkohol mit Natrium- oder Kaliumalkoholaten zu Alkoxy-benzaldehyden oder Alkoxy-benzylalkoholen umsetzen lassen. Die Aldehyde liefern unter den erfindungsgemäßen Reaktionsbedingungen lediglich o- bzw. p-Chlor-benzylalkohol und o- bzw. p-Chlor-benzoesäure durch Cannizzaro-Reaktion oder reagieren mit den Lösungsmitteln Dimethylsulfoxid oder N-Methyl-pyrrolidon, während die Benzylalkohole unumgesetzt zurückgewonnen werden.

HR 104

## Beispiel 1

a) p-Chlor-benzaldehyd-ethylenacetal:

In einem mit Rührer, Innenthermometer und aufgesetztem Wasserabscheider versehenen 2 l-Dreihalskolben erhitzt man 562 g (4,0 mol) p-Chlor-benzaldehyd, 289 g (4,8 mol) Ethylenglykol und 0,4 g p-Toluol-sulfonsäure in 600 ml Toluol, bis sich kein Reaktionswasser mehr abscheidet (etwa 8 h). Danach wäscht man die Reaktionslösung einmal mit 100 g 5 %iger Natronlauge, entfernt das Toluol im Vakuum und destilliert den Rückstand im Vakuum.

Es werden 692 g (= 93,8 % der Theorie) p-Chlor-benzaldehyd-ethylenacetal als klare, farblose Flüssigkeit vom Kp 91-92°C/0,7 mbar erhalten.

b) p-Methoxy-benzaldehyd-ethylenacetal (Anisaldehyd-ethylenacetal):

Zu der in einem 6 l-Dreihalskolben vorgelegten Suspension von 178,2 g (3,3 mol) Natriummethylat in 2376 g (24 mol) trockenem N-Methyl-pyrrolidon werden unter Rühren bei Raumtemperatur innerhalb von 10 Min. 553,5 g (3,0 mol) p-Chlor-benzaldehyd-ethylenacetal getropft. Die Reaktionsmischung wird unter Rühren 16 h auf 125°C erhitzt. Anschließend wird zunächst im Vakuum das N-Methyl-pyrrolidon abdestilliert. Der Rückstand wird mit 1200 g Toluol und 1200 g warmem

HR 104

Wasser versetzt. Man rührt kräftig durch, trennt die organische Phase ab, extrahiert die wäßrige Phase mit Toluol und wäscht die vereinigten organischen Phasen mit Wasser. Dann entfernt man das Toluol im Vakuum und destilliert den Rückstand im Vakuum.

Man erhält 474 g Destillat, das bei 90-115°C/0,6 mbar übergeht und gemäß gaschromatographischer Analyse zu 71,5 Gew.-% aus p-Methoxy-benzaldehyd-ethylenacetal und 28,5 Gew.-% nicht umgesetztem p-Chlor-benzaldehyd-ethylenacetal besteht.

Durch fraktionierte Destillation des Destillats an einer 1 m-Füllkörperkolonne werden 338 g (= 82,6 % der Theorie, bezogen auf umgesetztes p-Chlor-benzaldehyd-ethylenacetal) reines p-Methoxy-benzaldehyd-ethylenacetal und 134 g nicht umgesetztes p-Chlor-benzaldehyd-ethylenacetal erhalten.

c) <u>p-Methoxy-benzaldehyd (Anisaldehyd)</u>:

180 g (1,0 Mol) p-Methoxy-benzaldehyd-ethylenacetal, 406 g (7,0 mol) Aceton und 0,5 g p-Toluolsulfonsäure werden 2 h bei 25°C gerührt und dann mit 1,0 g Natriumcarbonat versetzt. Man destilliert zunächst das überschüssige Aceton und dann das entstandene Acetonethylenketal (Kp: 92°C) bei Normaldruck ab und destilliert danach im Vakuum den Rückstand. Es werden 130 g (= 95,6 % der Theorie) Anisaldehyd vom Kp 94-95°C/4 mbar erhalten.

<u>HR 104</u>

Beispiel 2

a) <u>p-Chlor-benzaldehyd-di-n-pentyl-acetal</u>:

In einem mit Rührer, Innenthermometer und aufgesetztem Wasserabscheider versehenen 4 l-Dreihalskolben erhitzt man 843 g (6,0 mol) p-Chlor-benzaldehyd, 1162 g (13,2 mol) n-Pentanol und 18 g p-Toluol-sulfonsäure in 700 ml Toluol, bis sich kein Reaktionswasser mehr abscheidet (etwa 16 h). Danach kühlt man das Reaktionsgemisch auf Raumtemperatur ab, gibt 12 g 50 %ige Natronlauge zu und destilliert das Toluol im Vakuum ab. Bei der Destillation des Rückstandes im Vakuum werden 1190 g (66,5 % der Theorie) p-Chlor-benzaldehyd-di-n-pentyl-acetal vom Kp 130-135°C/ 0,7 mbar erhalten.

b) <u>p-Methoxy-benzaldehyd-di-n-pentylacetal (Anisaldehyd-di-n-pentylacetal)</u>:

Zu einer Suspension von 54 g (1,0 mol) Natriummethylat in 396 g (4,0 mol) trockenem N-Methyl-pyrrolidon werden unter Rühren innerhalb von 10 Min 298,5 g (1,0 mol) p-Chlor-benzaldehyd-di-n-pentylacetal getropft. Die Reaktionsmischung wird 20 h bei 140°C gerührt. Anschließend wird die Reaktionsmischung, wie im Beispiel 1 b) beschrieben, aufgearbeitet.

Die Destillation ergibt 214 g Destillat, das bei 134-144°C/2,0 mbar übergeht und gemäß gaschromatographi-

<u>HR 104</u>

- 12 -

scher Analyse aus 63,6 Gew.-% p-Methoxy-benzaldehyd-
di-n-pentylacetal und 36,4 Gew.-% nicht umgesetztem
p-Chlor-benzaldehyd-di-n-pentylacetal besteht.

Die fraktionierte Destillation des Destillats liefert
136 g (= 62,3 % der Theorie, bezogen auf umgesetztes
p-Chlor-benzaldehyd-di-n-pentylacetal) p-Methoxybenzaldehyd-di-n-pentylacetal und 77 g nicht umgesetztes
p-Chlor-benzaldehyd-di-n-pentylacetal.

c) <u>p-Methoxy-benzaldehyd (Anisaldehyd)</u>:

117,6 g (0,4 mol) p-Methoxy-benzaldehyd-di-n-pentyl-
acetal, 150 g 5-prozentige Schwefelsäure und 150 g
Eisessig werden 6 h unter Rückfluß erhitzt. Das
Reaktionsgemisch wird abgekühlt, die organische Phase
abgetrennt und die wäßrige Phase mit Toluol extrahiert. Die vereinigten organischen Phasen werden
mit Wasser gewaschen, vom Toluol befreit und destilliert. Man erhält 50,5 g (= 92,8 % der Theorie)
p-Methoxy-benzaldehyd.

<u>Beispiel 3</u>

a) <u>p-Methoxy-benzaldehyd-ethylenacetal (Anisaldehyd-
ethylenacetal)</u>:

Zu einer Suspension von 237,6 g (4,4 mol) Natriummethylat in 2496 g (32 mol) trockenem Dimethylsulfoxid werden unter Rühren innerhalb von 10 Min.
738 g (4 mol) gemäß Beispiel 1 a) hergestelltes
p-Chlor-benzaldehyd-ethylenacetal getropft. Die
Reaktionsmischung wird 16 h bei 100°C gerührt.
Anschließend wird das Dimethyl

HR 104

- 13 -

sulfoxid im Vakuum abdestilliert. Der Rückstand wird, wie in Beispiel 1 b) beschrieben, aufgearbeitet. Es werden 408 g (= 90,1 % der Theorie, bezogen auf umgesetztes p-Chlor-benzaldehyd-ethylenacetal) p-Methoxy-benzaldehyd-ethylenacetal vom Kp 112°C/0,6 mbar und 274 g nicht umgesetztes p-Chlor- benzaldehyd-ethylenacetal vom Kp 90°C/0,6 mbar erhalten.

b)   p-Methoxy-benzaldehyd (Anisaldehyd):

360 g (2 mol) p-Methoxy-benzaldehyd-ethylenacetal und 600 g 5 %ige Schwefelsäure werden 6 h unter Rückfluß erhitzt. Die Reaktionsmischung wird abgekühlt, die organische Phase abgetrennt und die wäßrige Phase mit Toluol extrahiert. Die vereinigten organischen Phasen werden mit Wasser gewaschen, vom Toluol befreit und destilliert. Man erhält 256 g (94,1 % der Theorie) Anisaldehyd vom Kp 94-95°C/4 mbar.

Beispiel 4

a)   p-Methoxy-benzaldehyd-ethylenacetal (Anisaldehydethylenacetal):

Zu einer Suspension von 70 g (1,0 mol) Kaliummethylat in 297 g (3,0 mol) trockenem N-Methyl-pyrrolidon werden unter Rühren innerhalb von 10 Min. 138 g (0,75 mol) gemäß Beispiel 1 a) hergestelltes p-Chlor-benzaldehyd- ethylenacetal getropft. Die Reaktionsmischung wird 20 h bei 110°C gerührt. Anschließend wird sie, wie in Beispiel 1 b) beschrieben, aufgearbeitet.

HR 104

Man erhält 97 g Destillat vom Kp 95-115°C/1mbar, das gemäß gaschromatographischer Analyse aus 84,1 Gew.-% p-Methoxy-benzaldehyd-ethylenacetal und 15,9 Gew.-% unumgesetztem p-Chlor-benzaldehyd-ethylenacetal besteht.

Durch fraktionierte Destillation des Destillates werden 81 g (= 67,5 % der Theorie, bezogen auf umgesetztes p-Chlor-benzaldehyd- ethylenacetal) p-Methoxy-benzaldehyd-ethylenacetal vom Kp 112°C/0,6 mbar und 15 g unumgesetztes p-Chlor-benzaldehyd-ethylenacetal vom Kp 90°C/0,6 mbar erhalten.

b)    p-Methoxy-benzaldehyd (Anisaldehyd):

Die Verseifung des p-Methoxy-benzaldehyd-ethylenacetals wird, wie in Beispiel 1 c) beschrieben, vorgenommen. Es werden 57 g (= 93,1 % der Theorie) Anisaldehyd vom Kp 94-95°C/4 mbar erhalten.

Beispiel 5

a)    o-Chlor-benzaldehyd-ethylenacetal:

562 g (4,0 mol) o-Chlor-benzaldehyd, 298 g (4,8 mol) Ethylenglykol und 0,4 g p-Toluol-sulfonsäure in 600 ml Toluol werden, wie in Beispiel 1 a) beschrieben, umgesetzt.

HR 104

Es werden 701 g (= 95,0 % der Theorie) o-Chlor-benzaldehyd-ethylenacetal als klare, farblose Flüssigkeit vom Kp 85 - 87°C/1,5 mbar erhalten.

b)   o-Methoxy-benzaldehyd-ethylenacetal

Zu der in einem 1 l-Dreihalskolben vorgelegten Suspension von 59,4 g (1,1 mol) Natriummethylat in 396 g (4,0 mol) trockenem N-Methyl-pyrrolidon werden unter Rühren innerhalb von 10 Min. 184,5 g (1,0 mol) o-Chlor-benzaldehyd-ethylenacetal getropft. Die Reaktionsmischung wird 15 h bei 110°C gerührt und anschließend, wie in Beispiel 1 b) beschrieben, aufgearbeitet.

Man erhält 168 g Destillat (Kp 78-85°C/0,9 mbar), das gemäß gaschromatographischer Analyse zu 51,2 Gew.-% aus o-Methoxy-benzaldehyd-ethylenacetal und 48,8 Gew.-% aus o-Chlor-benzaldehyd-ethylenacetal besteht.

Die fraktionierte Destillation des Destillates liefert 86 g (= 85,2 % der Theorie, bezogen auf umgesetztes o-Chlor-benzaldehyd-ethylenacetal) o-Methoxy-benzaldehyd-ethylenacetal mit einem Kp 93°C/1,5 mbar und 81 g unumgesetztes o-Chlor-benzaldehyd-ethylenacetal (Kp 85°C/1,5 mbar).

HR 104

c) <u>o-Methoxy-benzaldehyd</u>:

72 g (0,4 mol) o-Methoxy-benzaldehyd-ethylenacetal und 120 g 5-prozentige Schwefelsäure werden 6 h unter Rückfluß erhitzt. Das Reaktionsgemisch wird abgekühlt, die organische Phase abgetrennt und die wäßrige Phase mit Toluol extrahiert. Die vereinigten organischen Phasen werden mit Wasser gewaschen, vom Toluol befreit und destilliert. Man erhält 51 g (= 93,8 % der Theorie) o-Methoxy-benzaldehyd vom Kp 95°C/4,0 mbar.

<u>Beispiel 6</u>

a) <u>p-n-Octyloxy-benzaldehyd-ethylenacetal</u>:

Zur Lösung von 76 g (0,5 mol) Natrium-n-octanolat in 396 g (4,0 mol) trockenem N-Methyl-pyrrolidon werden unter Rühren innerhalb von 10 Min. 92 g (0,5 mol) gemäß Beispiel 1 a) hergestelltes p-Chlor-benzaldehyd-ethylenacetal getropft. Die Reaktionsmischung wird 16 h bei 125°C gerührt und anschließend, wie in Beispiel 1 b) beschrieben, aufgearbeitet.

Bei der fraktionierten Destillation erhält man 60 g (= 69,7 % der Theorie, bezogen auf umgesetztes p-Chlor-benzaldehyd-ethylenacetal) Octyloxy-benzaldehyd-ethylenacetal (Kp 144°C/1 mbar) und 35 g nicht umgesetztes p-Chlor-benzaldehyd-ethylenacetal (Kp 98°C/1 mbar).

<u>HR 104</u>

b)  p-n-Octyloxy-benzaldehyd:

Man erhitzt 41,7 g (0,15 mol) p-n-Octyloxy-benzaldehyd-ethylenacetal, 50 g 5-prozentige Schwefelsäure und 50 g Eisessig 5 h unter Rückfluß und
arbeitet wie in Beispiel 5c) beschrieben auf. Man
erhält 32 g (=91,2 % der Theorie) p-n-Octyloxy-benzaldehyd vom Kp 123-125°C/0,7 mbar.

Beispiel 7

a)  p-Isopropyloxy-benzaldehyd-ethylenacetal:

Man erhitzt in einem 1 l-Dreihalskolben 92 g
(0,5 mol) gemäß Beispiel 1 a) hergestelltes p-Chlor-
benzaldehyd-ethylenacetal und 41 g (0,5 mol) Natriumisopropylat in 396 g (4,0 mol) N-Methyl-pyrrolidon
20 h auf 130°C. Die Reaktionsmischung wird, wie in
Beispiel 1 b) beschrieben, aufgearbeitet.

Man erhält 14 g (= 25,3 % der Theorie, bezogen auf
umgesetztes p-Chlor-benzaldehyd-ethylenacetal) p-Isopropyloxy-benzaldehyd-ethylenacetal
(Kp 95°C/0,2 mbar) und 43 g nicht umgesetztes
p-Chlor-benzaldehyd-ethylenacetal.

HR 104

b) p-Isopropyloxy-benzaldehyd:

Man erhitzt 10,4 g (0,05 mol) p-Isopropyloxy-benzaldehyd-ethylenacetal, 20 g 5-prozentige Schwefelsäure
und 20 g Eisessig 5 h unter Rückfluß und arbeitet
wie in Beispiel 5c) beschrieben auf. Man erhält 7,4 g
(90,2 % der Theorie) p-Isopropyloxy-benzaldehyd vom
Kp 85-87°C/2,0 mbar.

HR 104

**Patentansprüche:**

1.  Verfahren zur Herstellung von o- bzw. p-Alkoxy-benzaldehyden, dadurch gekennzeichnet, daß man

    a)  o- bzw. p-Chlor-benzaldehyd in an sich bekannter
        Weise mittels eines einwertigen Alkohols, eines
        Diols oder eines Orthoesters in ein o- bzw. p-
        Chlor-benzaldehyd-acetal überführt,

    b)  dieses o- bzw. p-Chlor-benzaldehyd-acetal durch
        Umsetzen mit einem Natrium- oder Kaliumalkoholat
        in einem aprotischen, polaren organischen Lösungsmittel bei erhöhten Temperaturen in ein o-
        bzw. p-Alkoxy-benzaldehyd-acetal überführt und

    c)  dieses o- bzw. p-Alkoxy-benzaldehyd-acetal in
        an sich bekannter Weise durch sauer katalysierte
        Hydrolyse oder Umacetalisierung in den entsprechenden o- bzw. p-Alkoxy-benzaldehyd überführt.

2.  Verfahren nach Anspruch 1, dadurch gekennzeichnet,
    daß in Stufe (a) zur Acetalisierung Ethylenglykol
    verwendet wird.

3.  Verfahren nach Anspruch 1 und 2, dadurch gekennzeichnet, daß in Stufe (b) als Alkalialkoholat Natrium-
    methylat verwendet wird.

HR 104

4. Verfahren nach Ansprüchen 1 bis 3, dadurch gekennzeichnet, daß in Stufe b) als aprotisches, polares organisches Lösungsmittel Dimethylsulfoxid oder N-Methyl-pyrrolidon verwendet werden.

HR 104